# EUROPÄISCHE PATENTANMELDUNG

(11) **EP 0 838 591 A1**
(43) Veröffentlichungstag der Anmeldung: **29.04.1998**
(21) Anmeldenummer: 96117160.0
(22) Anmeldetag: 25.10.1996
(51) Int. Cl.: F04B 45/04, F04B 43/00

(54) **Inhalationsgerätekompressor mit verbessertem Membransatz**

(71) Anmelder: PARI GmbH Spezialisten für effektive Inhalation, 82319 Starnberg (DE)
(72) Erfinder: Brugger, Stephan, 82301 Starnberg (DE); Hertl, Erich, 82205 Gilching (DE); Lintl, Andreas, 82319 Starnberg (DE); Remke, Matthias, 82319 Starnberg (DE)
(74) Vertreter: Zangs, Rainer E., Dipl.-Ing.

(57) **Zusammenfassung**

Ein Membransatz für die Membranpumpe eines Inhalationsgerätekompressors enthält eine flexible Membran 1, 21 mit einer ersten Öffnung 2, 22, einen unteren Membranteller 6, 26 mit einer zweiten Öffnung 8, 28 und einen oberen Membranteller 14, 38 mit einer dritten Öffnung 16, 40, wobei die Membranteller 6, 14, 26, 38 einen geringeren Durchmesser als die flexible Membran 1, 21 aufweisen, und ein Befestigungselement 18, 44 zum Einspannen der Membran 1, 21 zwischen dem unteren Membranteller 6, 26 und dem oberen Membranteller 14, 38, wobei das Befestigungselement 18, 44 durch die erste, zweite und dritte Öffnung 2, 12, 16, 22, 28, 40 geführt ist und mit einer Membran-Antriebsvorrichtung im Eingriff steht. Zur Verbesserung des Betriebsverhaltens und der Herstellbarkeit wird vorgeschlagen am Außenumfang der flexiblen Membran 1, 21 einen streifenfömiger Abschnitt 4, 24 zu der Achse des Befestigungselementes 18, 44 hin anzuwinkeln und entlang des oberen Randes der zweiten Öffnung 8, 28 einen Anschlag 10, 30 zu bilden, an dem der Innenrand der ersten Öffnung der flexiblen Membran 1, 21 anliegen kann.

## Beschreibung

Die vorliegende Erfindung betrifft einen Membransatz für eine Membranpumpe eines Inhalationsgerätekompressors mit den Merkmalen des Oberbegriffs des Patentanspruchs 1.

Ein derartiger Membransatz ist aus DE 86 26 979.9 bekannt. Wie in Fig. 7 gezeigt ist, wird eine ebene Membran 100 zwischen einem oberen Membranteller 101 und einem unteren Membranteller 102 eingespannt. Dies erfolgt mit Hilfe einer Senkschraube 103, die durch Öffnungen in der Mitte des oberen Membrantellers, der Membran, und des unteren Membrantellers geführt wird, so daß sie mit einer Pleuelstange zum Betätigen der Membran verschraubt werden kann. Überlicherweise wird zwischen dem unteren Membranteller und der Pleuelstange ein Zwischenring 104 eingefügt.

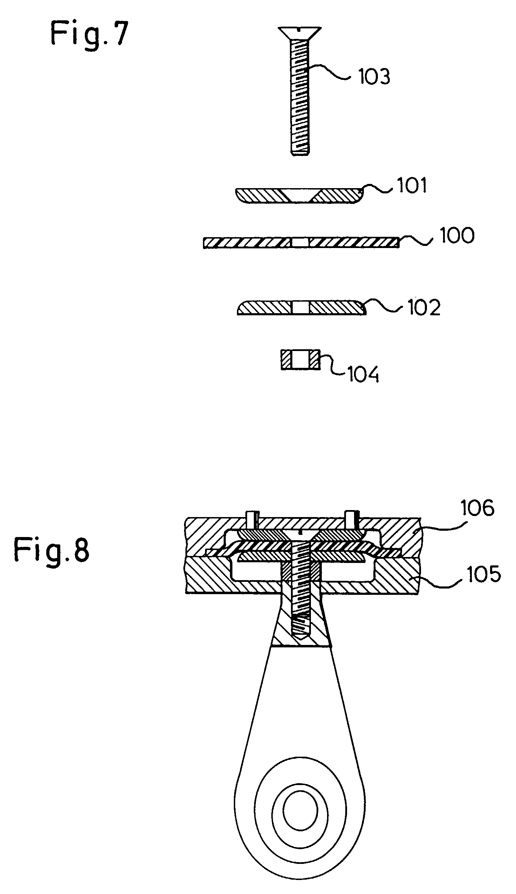

Wie in Fig. 8 gezeigt ist, kommt ein derartiger Membransatz in einem Membrankompressor zum Einsatz, der aus einem Verdichtergehäuse mit einem Gehäuseunterteil 105 und einem Gehäuseoberteil 106 aufgebaut ist. Im Gehäuseoberteil 106 ist eine umlaufende Nut vorgesehen, die den äußeren Rand der flexiblen Membran aufnimmt. Die Membran wird durch Pressen zwischen dem Gehäuseunterteil und dem Gehäuseoberteil in ihrer Lage innerhalb der Nut festgehalten. Die beiden Membranteller, die an der Ober- und Unterseite der Membran anliegen, sind verwindungssteif, so daß die zwischengelegte Membran lediglich in ihrem freien Randbereich elastisch verformbar ist.

Derartige Membransätze werden zum Fördern und Verdichten von flüssigen und gasförmigen Medien eingesetzt, beispielsweise in Inhaliergeräten. In der Regel wird hierbei das Konstruktionsziel verfolgt, einen möglichst großen Einsatzbereich abzudecken, so daß bei fest vorgegebener Antriebsleistung ein möglichst großer Druck und hoher Durchsatz des strömenden Mediums erreicht werden muß. Dies wird üblicherweise durch eine Anpassung des Totraums unmittelbar über dem eingespannten Rand der Membran und durch eine Vergrößerung des nicht versteiften Randbereichs der flexiblen Membran erreicht.

Durch den vergrößerten Randbereich der flexiblen Membran erhöht sich jedoch die Geräuschentwicklung, was insbesondere bei der Anwendung in Inhaliergeräten kritisch ist. Hier muß darauf geachtet werden, daß der Therapieeffekt bei dem zu behandelnden Patienten nicht durch eine übermäßig hohe Geräuschentwicklung beeinträchtigt wird.

Zudem weist der Membransatz nach dem Stand der Technik, wie er in Fig. 7 gezeigt ist, nicht unerbliche Probleme im Hinblick auf die Herstellung auf, die bisher ungelöst sind. Wird, wie oben erläutert, die Membran zwischen dem oberen Membranteller und dem unteren Membranteller mit Hilfe einer Senkschraube eingespannt, so ergeben sich in Abhängigkeit der Kraft, mit der die Senkschraube angezogen wird, unterschiedliche Radien der Membran nach dem Einspannen und damit unterschiedliche Vorspannungen im Membranmaterial.

Zudem ist es schwierig, ein gleichmäßiges Einspannen der flexiblen Membran bei unterschiedlichen Membransätzen zu gewährleisten, und es treten hohe Toleranzen bei der Herstellung der Membransätze auf. Insbesondere in den Fällen, in denen die Membran besonders fest einzuspannen ist, muß die Schraube mit hoher Kraft angezogen werden, was für das Personal besonders anstrengend und ermüdend ist. Diese Vorgehensweise ist insgesamt umständlich und zeitaufwendig, so daß hier aufgrund der zunehmenden Stückzahlen, mit denen derartige Membransätze gefertigt werden ein akuter Handlungsbedarf besteht.

Ferner ist in US-A-3 021 792 eine Benzinpumpe beschrieben, mit der ein möglichst hoher Volumendurchsatz pro Zeiteinheit erzielt werden soll. Es wird vorgeschlagen, den im oberen Totpunkt der Membran innerhalb der Benzinpumpe verbleibenden freien Bereich zu minimieren. Die Membran ist nicht vorgeformt und somit vor dem Einbau völlig flach ausgebildet, so daß auch hier die oben genannten Nachteile bestehen.

Die der vorliegenden Erfindung zugrundeliegende Aufgabe besteht darin, einen Membransatz zu schaffen, der sich bei verbesserten Herstellungstoleranzen einfach herstellen läßt und gleichzeitig günstige Betriebseigenschaften und eine lange Lebensdauer aufweist.

Diese Aufgabe wird erfindungsgemäß bei einem Membransatz für eine Membranpumpe der eingangs genannten Art durch die kennzeichnenden Merkmale des Patentanspruchs 1 gelöst.

Ein wichtiger Vorteil der Erfindung besteht darin, daß durch das Anwinkeln des Randabschnitts der flexiblen vorgeformten Membran bereits von vorneherein eine Formgebung der flexiblen vorgeformten Membran erreicht wird, die für einen ordnungsgemäßen Betrieb des Membransatzes erforderlich ist, so daß beim Einspannen der flexiblen vorgeformten Membran zwischen dem oberen und unteren Membranteller erheblich geringere Kräfte aufgebracht werden müssen. Im Ergebnis wird das Personal entlastet und der Herstellungsprozeß beschleunigt.

Ein weiterer wichtiger Vorteil der Erfindung besteht darin, daß die flexible vorgeformte Membran an der Innenseite ihrer Öffnung an einem Anschlag anliegt. Dies führt dazu, daß beim Ausrichten des oberen und unteren Membrantellers sowie der flexiblen vorgeformten Membran praktisch keine Schwankungen mehr auftreten können und sich die Herstellungstoleranzen erheblich verbessern lassen.

Wird, wie oben beschrieben, der äußere Rand der flexiblen, vorgeformten Membran in eine Nut des Gehäuseoberteils zwischen dem Gehäuseoberteil und dem Gehäuseuntereil eingepreßt, so ragt der nicht versteifte Randbereich der flexiblen, vorgeformten Membran wulstförmig in den Totraum hinein, wodurch dieser verringert wird. Demnach ermöglicht der erfindungsgemäße Membransatz ein verbessertes Geräuschverhalten, da durch den verringerten Totraum insgesamt weniger Störgeräusche entstehen.

Auch kann der wulstförmig in den Totraum des Membrankompressors hineinragende nicht versteifte Randbereich der flexiblen, vorgeformten Membran bei einer Auf- und Abbewegung der flexiblen, vorgeformten Membran direkt abrollen. Durch diesen Abrollvorgang läßt sich die bei der Auf- und Abbewegung auftretende Verformung in der flexiblen, vorgeformten Membran herabsetzen, so daß sich die Lebensdauer der flexiblen, vorgeformten Membran erheblich verlängert.

Zusätzlich bewirkt der wulstförmige Verlauf des nicht versteiften Randbereichs der flexiblen, vorgeformten Membran eine kontinuierliche Veränderung des Totraums, was dazu führt, daß sich der Betriebsdruck beim Verdichten von flüssigen oder gasförmigen Medien erhöht und sich der Maximaldruck während des Betriebs erniedrigt. Dadurch ergibt sich zudem ein flacher Kennlinienverlauf.

Bei einer weiteren, besonders bevorzugten Ausführungsform des erfindungsgemäßen Membransatzes ist bei dem unteren Membranteller zwischen dem Außenumfang und dem Anschlag mindestens eine Zentrierungsnut für die vorgeformte Membran vorgesehen, wobei die vorgeformte Membran in entsprechender Weise mindestens einen Haltering aufweist. Bei dieser Ausführungsform wird die Ausrichtung der einzelnen Bauteile des Membransatzes zueinander weiter verbessert, da unabhängig von der Kraft, mit der die flexible, vorgeformte Membran zwischen dem oberen und dem unteren Membranteller eingespannt wird, ein Verformen der flexiblen, vorgeformten Membran im Bereich der Einspannstelle ausgeschlossen ist.

In einer besonders vorteilhaften Weiterbildung des erfindungsgemäßen Membransatzes ist vorgesehen, daß der untere Membranteller an seinem Außenumfang einen Wulst aufweist. Dies begünstigt in vorteilhafter Weise den oben beschriebenen Abrollvorgang bei der Auf- und Abwärtbewegung der flexiblen, vorgeformten Membran.

Weitere Merkmale und Vorteile der Erfindung ergeben sich aus der folgenden Beschreibung mehrerer Ausführungsbeispiele, die in der Zeichnung dargestellt sind; es zeigen:
- Fig. 1: einen Querschnitt durch die erste Ausführungsform des erfindungsgemäßen Membransatzes in Explosionsdarstellung;
- Fig. 2: einen Querschnitt durch die erste Ausführungsform des erfindungsgemäßen Membransatzes in montierter Form;
- Fig. 3: einen Querschnitt durch die zweite Ausführungsform des erfindungsgemäßen Membransatzes in Explosionsdarstellung;
- Fig. 4: eine teilweise Querschnittsansicht durch die zweite Ausführungsform des erfindungsgemäßen Membransatzes in montierter Form;
- Fig. 5: eine Draufsicht auf eine Ausführungsform eines erfindungsgemäßen unteren Membrantellers;
- Fig. 6a bis 6c: jeweils eine Querschnittsansicht der ersten Ausführungsform des erfindungsgemäßen Membransatzes nach dem Einbau in einen Membrankompressor entsprechend einer unteren, mittleren und oberen Betriebsposition;
- Fig. 7: eine Querschnittsansicht durch einen embransatz entsprechend dem Stand der Technik in Explosionsdarstellung; und
- Fig. 8: eine Querschnittsansicht durch einen Membrankompressor entsprechend dem Stand der Technik mit dem bekannten Membransatz.

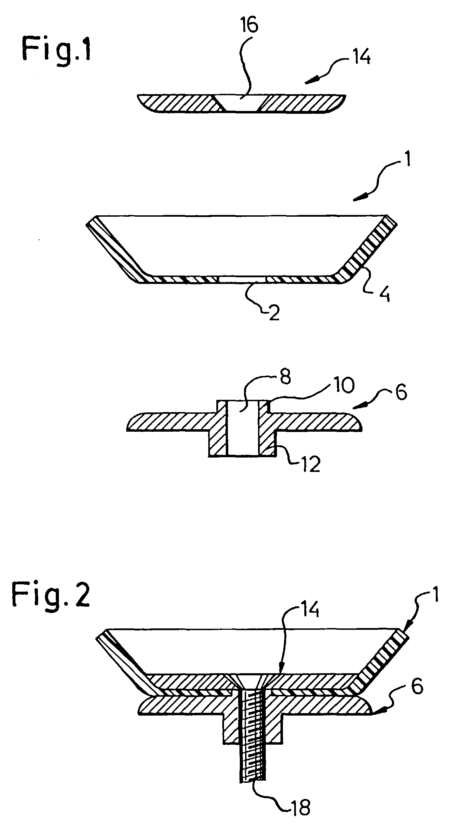

Eine erste Ausführungsform des erfindungsgemäßen Membransatzes ist in Fig. 1 gezeigt. Eine flexible Membran 1 weist in ihrer Mitte eine erste Öffnung 2 auf. Weiterhin ist die flexible Membran im wesentlichen kegelstumpfförmig, also vorgeformt, ausgebildet, so daß an ihrem Außenumfang ein streifenförmiger Abschnitt 4 nach innen angewinkelt ist. Die flexible Membran 1 wird an ihrer Unterseite von einem unteren Membranteller 6 mit einer zweiten Öffnung 8 gehalten. Der Durchmesser des unteren Membrantellers 6 wird so gewählt, daß sich dieser bis zu dem streifenförmigen Abschnitt 4 der flexiblen Membran 1 erstreckt. Zudem ist entlang des oberen Randes der zweiten Öffnung 8 ein Anschlag 10 gebildet, an dem der Innenrand der ersten Öffnung 2 der flexiblen Membran 1 anliegt. Entlang des unteren Randes der zweiten Öffnung 8 des unteren Membrantellers 6 ist ein Ansatzabschnitt 12 gebildet, so daß der untere Membranteller 6 insgesamt einen hohlzylinderartigen Abschnitt flanschartig umgibt.

Die flexible Membran 1 wird an ihrer Oberseite durch einen oberen Membranteller 14 gehalten, dessen Durchmesser im wesentlichen mit dem Durchmesser des unteren Membrantellers übereinstimmt. Der obere Membranteller 14 weist in seiner Mitte eine dritte Öffnung 16 auf.

Durch die erste, zweite und dritte Öffnung 2, 8, 16 kann ein Befestigungselement 18 so durchgeführt werden, daß die flexible Membran zwischen dem oberen Membranteller 14 und dem unteren Membranteller 6 eingespannt wird, wie in Fig. 2 gezeigt ist.

Es zeigt sich, daß durch das Anliegen der flexiblen Membran 1 an dem Anschlag 10 stets eine richtige Ausrichtung der einzelnen Teile zueinander möglich ist, ohne daß während der Herstellung besondere zusätzliche Vorkehrungen zu treffen sind. Zudem ist aufgrund der Tatsache, daß die flexible Membran stumpf kegelförmig mit einem seitlich angewinkelten streifenförmigen Abschnitt vorgeformt ist, eine Formgebung durch besonders festes Einspannen der flexiblen Membran 1 zwischen dem oberen Membranteller 14 und dem unteren Membranteller 6 nicht erforderlich.

Demnach ist bei der Herstellung des erfindungsgemäßen Membransatzes entsprechend der ersten Ausführungsform bereits eine niedrige Spannkraft beim Zusammenfügen der Bauteile ausreichend, so daß sich die stumpfe Kegelform der flexiblen Membran 1 hierbei nicht verändert. Im Ergebnis lassen sich erheblich geringere Herstellungstoleranzen einhalten. Aufgrund der geringeren Drehkräfte beim Zusammenfügen der Bauteile und bedingt durch den Anschlag 10 zum einfachen Ausrichten der Bauteile zueinander, wird das Personal zudem erheblich entlastet.

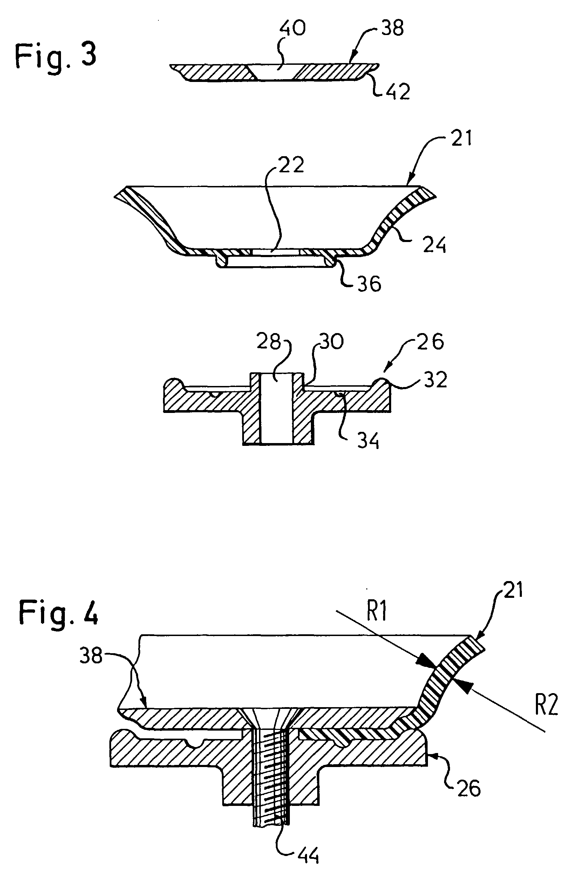

Eine zweite Ausführungsform des erfindungsgemäßen Membransatzes ist in den Fig. 3 und 4 gezeigt. Hier ist eine flexible Membran 21 im wesentlichen kelchförmig ausgebildet und in ihrer Mitte mit einer ersten Öffnung 22 versehen. Der streifenförmige Abschnitt 24 der flexiblen Membran 21 ist an seiner Oberseite mit einem ersten Radius R1 und an seiner Unterseite mit einem zweiten Radius R2 gekrümmt, wobei die beiden Radien in einem Verhältnis von etwa 1,125 stehen. Durch diese unterschiedliche Krümmung wird eine nach außen hin abgerundete Formgebung des streifenförmigen Abschnitts 24 der flexiblen Membran 21 erreicht.

An der Unterseite wird die flexible Membran 21 von einem unteren Membranteller 26 mit einer zweiten Öffnung 28 gehalten. Entlang des oberen Randes der zweiten Öffnung 28 ist ein Anschlag 30 gebildet, an dem der Innenrand der ersten Öffnung 22 der flexiblen Membran 21 anliegt. Zudem weist der untere Membranteller 26 an seinem Außenumfang einen Wulst 32 auf. Zwischen dem Wulst 32 und dem Anschlag 30 ist eine Zentriernut 34 vorgesehen, wobei die flexible Membran 21 an der entsprechenden Stelle mit einem kreisförmigen Haltevorsprung 36 ausgebildet ist.

Die flexible Membran 21 wird an der Oberseite durch einen oberen Membranteller 38 mit einer dritten Öffnung 40 gehalten. Der obere Membranteller 38 weist an der Unterseite seines Außenumfangs eine Rundausnehmung 42 auf, deren Krümmungsradius an den Außenradius des Wulst 32 des unteren Membrantellers 26 angepaßt ist.

Der obere Membranteller 38, die flexible Membran 21 und der untere Membranteller 26 werden durch ein Befestigungselement 44, beispielsweise eine Senkschraube, zusammengefügt.

Zusätzlich zu den bereits bei der ersten Ausführungsform erläuterten Vorteilen ermöglicht die zweite Ausführungsform eine praktisch fehlerfreie Ausrichtung der einzelnen Bauteile zueinander. Dies ist auf die zusätzlich vorgesehene Zentriernut 34 und den zusätzlichen Haltevorsprung 36 der flexiblen Membran 21 zurückzuführen.

Weiterhin führt der Wulst 32 des unteren Membrantellers 26 zusammen mit der Rundausnehmung 42 des oberen Membrantellers 38 und der Ausbildung des streifenförmigen Abschnitts 24 der flexiblen Membran 21 mit unterschiedlichen Krümmungsradien R1, R2 an der Ober- und Unterseite zu einer besonders vorteilhaften wulstartigen Ausprägung des nicht versteiften Randbereichs des Membransatzes.

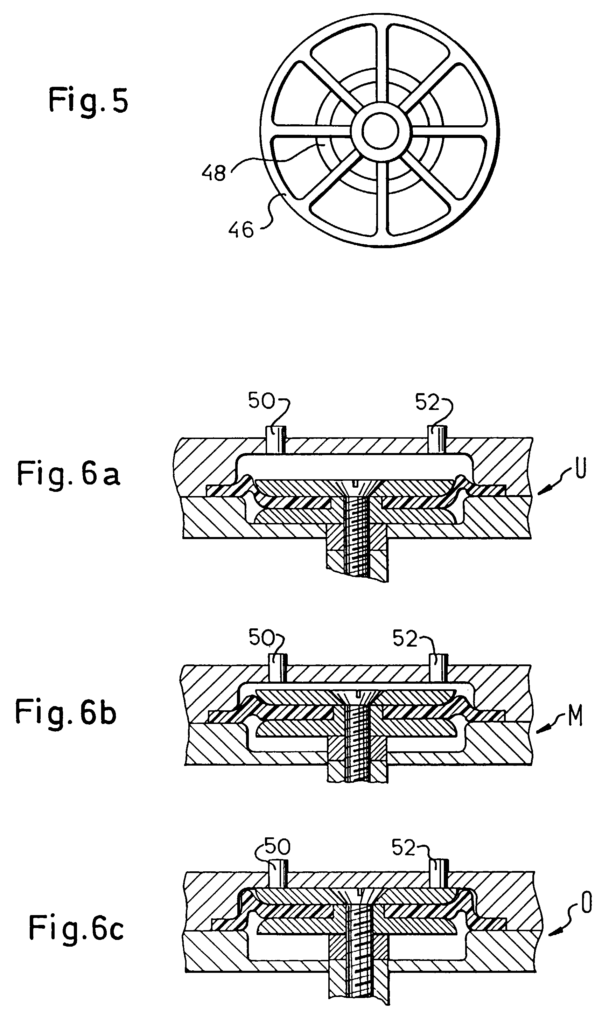

Wie in Fig. 5 gezeigt ist, kann der untere Membranteller zur Reduktion des Gewichts des Membransatzes als dünne Scheibe 46 ausgebildet sein, die zur Verbesserung der Formsteifigkeit mit einem zusätzlichen Versteifungsring 48 versehen ist. Durch das herabgesetzte Gewicht des Speichenrads 46 müssen beim Betrieb des Membransatzes geringere Massen bewegt werden. Insgesamt verringern sich die auftretenden Schwingungen und die hiermit verbundenen Störgeräusche, was insbesondere für die Anwendung des erfindungsgemäßen Membransatzes in Inhaliergeräten von besonderer Bedeutung ist.

Die Funktionsweise des erfindungsgemäßen Membransatzes soll im folgenden anhand der Fig. 6a bis 6c beschrieben werden.

Die flexible Membran 1 bzw. 21 des Membransatzes ist nach dem Einbau in einen Membrankompressor in einer Nut eines Gehäuseoberteils zwischen diesem Gehäuseoberteil und dem Gehäuseunterteil eingeklemmt. Wie Fig. 6a zeigt, ragt der nicht versteifte Randbereich der Membran 1, 21 wulstförmig in den Totraum des Membrankompressors hinein. Wie ein Vergleich der Fig. 6a, 6b und 6c zeigt, ist dies sowohl im unteren, als auch im mittleren und oberen Betriebspunkt U, M, O des Membransatzes der Fall.

Demnach wird die flexible Membran 1, 21 während des Betriebs nicht, wie aus dem Stand der Technik bekannt, durch die Aufund Abwärtsbewegung der Pleuelstange zum Antreiben des Membransatzes durchgehend verformt, sondern an die Stelle der Verformung tritt ein Abrollvorgang.

Dieser Abrollvorgang weist im praktischen Betrieb erhebliche Vorteile auf. Da die flexible Membran 1, 21 nicht fortlaufend durchgehend verformt wird, erhöht sich deren Lebensdauer. Zudem kann durch die erfindungsgemäße Ausformung der flexiblen Membran 1, 21 ein kontinuierlich veränderlicher Totraum erreicht werden. Dies führt zu einem erhöhten Betriebsdruck und einem reduzierten Maximaldruck für das zu komprimierende Medium, das während der Abwärtsbewegung des Membransatzes über eine Einlaßöffnung 50 in den Totraum angesaugt wird, um bei der anschließenden Aufwärtsbewegung des Membransatzes von einer unteren Betriebsposition U zu einer oberen Betriebsposition O durch die Auslaßöffnung 52 abgegeben zu werden.

Zusätzlich ersetzt der Abrollvorgang Hubraum, so daß eine größere Dimensionierung des Membransatzes und eine hierdurch bedingte größere Dimensionierung einer Membranpumpe vermieden wird. Dies ist insbesondere bei dem Einsatz des erfindungsgemäßen Membransatzes in einem Inhaliergerät, das mit möglichst geringen Abmessungen zu realisieren ist, von Bedeutung.

## Patentansprüche

1. Inhalationsgerätekompressor mit einer Membranpumpe, die einen Membransatz enthält, der
a) eine flexible Membran (1; 21) mit einer ersten Öffnung (2; 22),
b) einen unteren Membranteller (6; 26) mit einer zweiten Öffnung (8; 28) und einen oberen Membranteller (14; 38) mit einer dritten Öffnung (16; 40), wobei die Membranteller (6, 14; 26, 38) einen geringeren Durchmesser als die flexible Membran (1; 21) aufweisen,
c) ein Befestigungselement (18; 44) zum Einspannen der Membran (1; 21) zwischen dem unteren Membranteller (6; 26) und dem oberen Membranteller ( 14; 38), wobei das Befestigungselement (18; 44) durch die erste, zweite und dritte Öffnung (2, 12, 16; 22, 28, 40) geführt ist und mit einer Membran-Antriebsvorrichtung der Membranpumpe im Eingriff steht, umfaßt,
***dadurch gekennzeichnet, daß***
e) die flexible Membran vorgeformt ist und am Außenumfang einen zur Achse des Befestigungselementes (18; 44) hin angewinkelten streifenfömigen Abschnitt (4; 24) aufweist, und
f) entlang des oberen Randes der zweiten Öffnung (8; 28) ein Anschlag (10; 30) gebildet ist, an dem der Innenrand der ersten Öffnung der vorgeformten Membran (1; 21) anliegt.

2. Inhalationsgerätekompressor nach Anspruch 1,
***dadurch gekennzeichnet, daß***
- die vorgeformte Membran (1) im wesentlichen kegelstumpfförmig ausgebildet ist.

3. Inhalationsgerätekompressor nach Anspruch 1,
***dadurch gekennzeichnet, daß***
- die vorgeformte Membran (21) im wesentlichen kelchförmig ausgebildet ist.

4. Inhalationsgerätekompressor nach einem der Ansprüche 1 bis 3,
***dadurch gekennzeichnet, daß***
- als Befestigungselement (18; 44) eine Senkschraube vorgesehen ist, und die dritte Öffnung (16; 40) des oberen Membrantellers (14; 38) an die Form der Senkschraube durch eine Ansenkung angepaßt ist.

5. Inhalationsgerätekompressor nach einem der Ansprüche 1 bis 4,
***dadurch gekennzeichnet, daß***
- entlang des unteren Randes der zweiten Öffnung (8; 28) des unteren Membrantellers (6; 26) ein Ansatzabschnitt (12) gebildet ist.

6. Inhalationsgerätekompressor nach einem der Ansprüche 1 bis 5,
***dadurch gekennzeichnet, daß***
- der untere Membranteller (26) an seinem Außenumfang einen umlaufenden Wulst (32) aufweist.

7. Inhalationsgerätekompressor nach einem der Ansprüche 1 bis 6,
***dadurch gekennzeichnet, daß***
- bei dem unteren Membranteller (26) zwischen dem Außenumfang und dem Anschlag (30) mindestens eine Zentrierungsnut (34) für die vorgeformte Membran (21) vorgesehen ist, wobei die vorgeformte Membran entsprechend der mindestens einen Zentrierungsnut (34) mit mindestens einem Haltering (36) versehen ist.

8. Inhalationsgerätekompressor nach einem der Ansprüche 1 bis 7,
***dadurch gekennzeichnet*****, da*****ß***
- der obere Membranteller (38) an der Unterseite seines Außenumfangs eine Rundausnehmung(42) aufweist, deren Radius an den Krümmungsradius des Wulsts (32) der unteren Membranplatte angepaßt ist.

9. Inhalationsgerätekompressor nach einem der Ansprüche 1 bis 8,
***dadurch gekennzeichnet, daß***
- der streifenförmige Abschnitt (24) der vorgeformten Membran (21) an seiner Oberseite mit einem ersten Radius (R1) und an seiner Unterseite mit einem zweiten Radius (R2) gekrümmt ist, wobei die beiden Radien in einem Verhältnis von etwa 1,125 stehen.

10. Inhalationsgerätekompressor nach einem der Ansprüche 1 bis 9,
***dadurch gekennzeichnet, daß***
- der obere Membranteller (21) als Speichenrad (46) mit mindestens einem zusätzlichen Versteifungsring (48) gebildet ist.
